# EUROPEAN PATENT APPLICATION

(11) **EP 4 786 579 A1**
(43) Date of publication of application: **05.08.2026**
(21) Application number: 24872401.5
(22) Date of filing: 26.09.2024
(51) Int. Cl.: C12N 5/00, A23J 3/20, C12N 1/00, C12N 5/0775, C12P 21/02

(54) **METHOD FOR CULTURING ANIMAL CELLS**

(30) Priority: 26.09.2023 JP 2023163151
(71) Applicant: AJINOMOTO CO., INC., Chuo-ku Tokyo 104-8315 (JP)
(72) Inventor: YAMADA, Kei, Kawasaki-shi, Kanagawa 210-8681 (JP); OGAWA, Shimpei, Kawasaki-shi, Kanagawa 210-8681 (JP); NOGUCHI, Keiko, Kawasaki-shi, Kanagawa 210-8681 (JP); HIGUCHI, Takuya, Kawasaki-shi, Kanagawa 210-8681 (JP); KATAYAMA, Takahiro, Kawasaki-shi, Kanagawa 210-8681 (JP); OHASHI, Haruka, Kawasaki-shi, Kanagawa 210-8681 (JP); SAWAI, Anna, Kawasaki-shi, Kanagawa 210-8681 (JP); FUKADA, Hiroaki, Kawasaki-shi, Kanagawa 210-8681 (JP)
(74) Representative: Hoffmann Eitle
(86) International application number: PCT/JP2024/034439
(87) International publication number: WO 2025/070616

(57) **Abstract**

Provided are a method for culturing animal cells and related technology, wherein animal cells are cultured in the presence of an iron ionophore such as hinokitiol.

## Description

### TECHNICAL FIELD

The present invention relates to a method for culturing animal cells and related technology.

### BACKGROUND ART

For culturing animal cells, a medium containing serum, such as fetal bovine serum, is widely used. However, from the standpoints of safety, economy, and the like, the development of a serum-free medium that does not contain serum is desired. For example, serum-free media have been developed in which serum is replaced with alternative components such as growth factors.

Transferrin is sometimes used in the culturing of animal cells (Non-Patent Literature 1). Transferrin is known to bind to iron and contribute to its transport (Non-Patent Literature 2-3).

Furthermore, hinokitiol is known as an iron ionophore, which has the ability to bind to iron ions and increase the membrane permeability of iron ions (Non-Patent Literature 4).

### CITATION LIST

### NON-PATENT LITERATURE

Non-Patent Literature 1: Tatsuma Yao and Yuta Asayama. Animal-cell culture media: History, characteristics, and current issues. Reprod Med Biol. 2017 Apr; 16(2): 99-117.
Non-Patent Literature 2: Fang, X. et al. The molecular and metabolic landscape of iron and ferroptosis in cardiovascular disease. Nature Reviews 20, 7-23 (2023).
Non-Patent Literature3: Waldvogel-Abramowski, S. et al. Physiology of Iron Metabolism. Transfus. Med. Hemother. 41, 213-221 (2014).
Non-Patent Literature 4: Science;12 May 2017;Vol.356,Issue 6338;p.608-616; doi: 10.1126/science.aah3862

### SUMMARY OF INVENTION

### TECHNICAL PROBLEM

An object of the present invention is to provide a method for culturing animal cells and related technology.

### SOLUTION TO PROBLEM

The present inventors have found that animal cells can be cultured in the absence of serum by using an iron ionophore such as hinokitiol, thereby completing the present invention.

That is, the present invention can be exemplified as follows.
[1] A composition for animal cell culture, comprising an iron ionophore.
[2] The composition (specifically, according to [1]), which is a composition for improving the growth and/or promoting the differentiation of animal cells.
[3] The composition (specifically, according to [1] or [2]), which is a medium or a medium supplement.
[4] The composition (specifically, according to [3]), wherein the medium is a basal medium, a fed-batch medium, or a perfusion medium.
[5] The composition (specifically, according to any one of [1] to [4]), wherein the iron ionophore is at least one component selected from the group consisting of hinokitiol, α-thujaplicin, and γ-thujaplicin.
[6] The composition (specifically, according to any one of [1] to [5]), wherein the iron ionophore is hinokitiol.
[7] The composition (specifically, according to any one of [1] to [6]), further comprising iron.
[8] The composition (specifically, according to [7]), wherein the iron is an iron salt.
[9] The composition (specifically, according to any one of [1] to [8]), wherein the animal is a mammal, a bird, a fish, or a crustacean.
[10] The composition (specifically, according to any one of [1] to [9]), wherein the animal is an animal for meat production.
[11] The composition (specifically, according to any one of [1] to [10]), wherein the animal is a cow, a pig, or a chicken.
[12] The composition (specifically, according to any one of [1] to [9]), wherein the animal is a human.
[13] The composition (specifically, according to any one of [1] to [12]), wherein the cell is a muscle stem cell, an adipose precursor cell, a kidney-derived cell, or a T cell.
[14] The composition (specifically, according to any one of [1] to [13]), which is substantially free of serum.
[15] The composition (specifically, according to any one of [1] to [14]), which is substantially free of animal-derived albumin.
[16] The composition (specifically, according to any one of [1] to [15]), which is substantially free of transferrin.
[17] The composition (specifically, according to any one of [1] to [16]), which has a function of improving the growth of animal cells and/or a function of promoting the differentiation of animal cells.
[18] A method for producing a product, comprising a step of culturing animal cells in the presence of an iron ionophore.
[19] The method (specifically, according to [18]), wherein the product is cultured meat.
[20] A method for culturing animal cells, comprising a step of culturing animal cells in the presence of an iron ionophore.
[21] A method for improving the growth of animal cells, comprising a step of culturing animal cells in the presence of an iron ionophore.
[22] A method for promoting the differentiation of animal cells, comprising a step of culturing animal cells in the presence of an iron ionophore.
[23] The method (specifically, according to any one of [18] to [22]), wherein the iron ionophore is at least one component selected from the group consisting of hinokitiol, α-thujaplicin, and γ-thujaplicin.
[24] The method (specifically, according to any one of [18] to [23]), wherein the iron ionophore is hinokitiol.
[25] The method (specifically, according to any one of [18] to [24]), wherein the culturing is performed in the presence of iron.
[26] The method (specifically, according to [25]), wherein the iron is an iron salt.
[27] The method (specifically, according to any one of [18] to [26]), wherein the concentration of the iron ionophore in the medium during culturing is from 1 nM to 100 µM.
[28] The method (specifically, according to any one of [18] to [27]), wherein the concentration of the iron ionophore in the medium during culturing is from 10 nM to 10 µM.
[29] The method (specifically, according to [23] or [24]), wherein the concentration of hinokitiol in the medium during culturing is from 1 nM to 2.5 µM.
[30] The method (specifically, according to [23] or [24]), wherein the concentration of hinokitiol in the medium during culturing is from 10 nM to 1 µM.
[31] The method (specifically, according to [25] or [26]), wherein the concentration of iron in the medium during culturing is from 200 nM to 500 µM.
[32] The method (specifically, according to any one of [18] to [31]), wherein the animal is a mammal, a bird, a fish, or a crustacean.
[33] The method (specifically, according to any one of [18] to [32]), wherein the animal is an animal for meat production.
[34] The method (specifically, according to any one of [18] to [33]), wherein the animal is a cow, a pig, or a chicken.
[35] The method (specifically, according to any one of [18], [20] to [32]), wherein the animal is a human.
[36] The method (specifically, according to any one of [18] to [35]), wherein the cell is a muscle stem cell or an adipose precursor cell.
[37] The method (specifically, according to any one of [18], [20] to [35]), wherein the cell is a kidney-derived cell or a T cell.
[38] The method (specifically, according to any one of [18] to [37]), wherein the culturing is performed in a medium substantially free of serum.
[39] The method (specifically, according to any one of [18] to [38]), wherein the culturing is performed in a medium substantially free of animal-derived albumin.
[40] The method (specifically, according to any one of [18] to [39]), wherein the culturing is performed in a medium substantially free of transferrin.
[41] The method (specifically, according to any one of [18] to [40]), wherein, by culturing the animal cells in the presence of the iron ionophore, the growth of the animal cells is improved and/or the differentiation of the animal cells is promoted.

### ADVANTAGEOUS EFFECTS OF INVENTION

According to the present invention, animal cells can be cultured.

### BRIEF DESCRIPTION OF DRAWINGS

[Fig. 1] Fig. 1 is a diagram showing the proliferation of bovine muscle stem cells in a serum-free medium in the presence of hinokitiol. The vertical axis indicates the fold increase. The horizontal axis indicates the group number.
[Fig. 2] Fig. 2 is a diagram showing the proliferation of porcine subcutaneous preadipocytes (PSPA) in a serum medium or a serum-free medium in the presence of hinokitiol. The vertical axis indicates the fold increase. The horizontal axis indicates the group number.
[Fig. 3] Fig. 3 is a diagram showing the viable cell count of a HEK293-derived cell line subcultured in a suspension culture system in the presence of hinokitiol. Cell counting and subculturing were performed on days 3, 7, 10, 13, 17, and 20. The vertical axis indicates the cell count (×10⁶ cells/ml), and the horizontal axis indicates the elapsed days from the start of the culture (days).
[Fig. 4] Fig. 4 is a diagram showing the proliferation of CD8-positive human T cells in a serum-free medium in the presence of hinokitiol. The vertical axis indicates the fold increase. The horizontal axis indicates the group number.

### DESCRIPTION OF EMBODIMENTS

### <1> Active Ingredient

### <1-1> Active Ingredient

In the present invention, an iron ionophore is used as an active ingredient.

An iron ionophore is also referred to as an "active ingredient".

The active ingredient can be used for culturing animal cells. Specifically, animal cells can be cultured in the presence of the active ingredient.

By using the active ingredient, specifically by culturing animal cells in the presence of the active ingredient, the growth of animal cells may be improved, that is, an effect of improving the growth of animal cells may be obtained. This effect is also referred to as a "growth-improving effect". In other words, the active ingredient may have a function of improving the growth of animal cells. This function is also referred to as a "growth-improving function". That is, by culturing animal cells in the presence of the active ingredient, the growth of animal cells may be improved as compared with the case where animal cells are cultured in the absence of the active ingredient. The case where animal cells are cultured in the absence of the active ingredient includes the case where animal cells are cultured under the same conditions as in the case where animal cells are cultured in the presence of the active ingredient, except that the active ingredient is not used. The growth-improving effect may be obtained, for example, when animal cells are cultured under conditions where serum is substantially absent (that is, when animal cells are cultured in a medium substantially free of serum). The growth-improving effect may be obtained, for example, when animal cells are cultured under conditions where animal-derived albumin is substantially absent (that is, when animal cells are cultured in a medium substantially free of animal-derived albumin). The growth-improving effect may be obtained, for example, when animal cells are cultured under conditions where serum and animal-derived albumin are substantially absent (that is, when animal cells are cultured in a medium substantially free of serum and animal-derived albumin). The growth-improving effect may be obtained, for example, when the animal cells are MyoD+ cells (that is, cells expressing MyoD). The MyoD+ cells may be, for example, myoblasts. Improvement of growth includes an increase in the proliferation rate. The growth-improving effect can be confirmed by measuring and comparing the growth of animal cells (for example, the proliferation rate) in the presence and absence of the active ingredient.

By using the active ingredient, specifically by culturing animal cells in the presence of the active ingredient, the differentiation of animal cells may be promoted, that is, an effect of promoting the differentiation of animal cells may be obtained. This effect is also referred to as a "differentiation-promoting effect". In other words, the active ingredient may have a function of promoting the differentiation of animal cells. This function is also referred to as a "differentiation-promoting function". That is, by culturing animal cells in the presence of the active ingredient, the differentiation of animal cells may be promoted as compared with the case where animal cells are cultured in the absence of the active ingredient. The case where animal cells are cultured in the absence of the active ingredient includes the case where animal cells are cultured under the same conditions as in the case where animal cells are cultured in the presence of the active ingredient, except that the active ingredient is not used. The differentiation-promoting effect may be obtained, for example, when animal cells are cultured under conditions where serum is substantially absent (that is, when animal cells are cultured in a medium substantially free of serum). The differentiation-promoting effect may be obtained, for example, when animal cells are cultured under conditions where animal-derived albumin is substantially absent (that is, when animal cells are cultured in a medium substantially free of animal-derived albumin). The differentiation-promoting effect may be obtained, for example, when animal cells are cultured under conditions where serum and animal-derived albumin are substantially absent (that is, when animal cells are cultured in a medium substantially free of serum and animal-derived albumin). The differentiation-promoting effect may be obtained, for example, when the animal cells are PAX7+ cells (that is, cells expressing PAX7). The PAX7+ cells may be, for example, satellite cells. The satellite cells may be activated. An example of differentiation is muscle differentiation. Promotion of differentiation includes an increase in the differentiation rate and an increase in the ratio of differentiated cells. The differentiation-promoting effect can be confirmed by measuring and comparing the differentiation of animal cells (for example, the differentiation rate and the ratio of differentiated cells) in the presence and absence of the active ingredient. The differentiation of animal cells can be measured, for example, using the expression of a protein that serves as an index of differentiation as an index. For example, in muscle differentiation, Sprouty1, Pax7, MyoD, Myogenin, and Myosin heavy chain (MHC) may be expressed in this order as differentiation progresses (Manuel Schmidt et al. Adult stem cells at work: regenerating skeletal muscle. Cell Mol Life Sci. 2019 Jul;76(13):2559-2570.).

By using the active ingredient, specifically by culturing animal cells in the presence of the active ingredient, at least a growth-improving effect may be obtained.

By using the active ingredient, specifically by culturing animal cells in the presence of the active ingredient, a growth-improving effect and a differentiation-promoting effect may be obtained.

The active ingredient may be used, for example, in place of transferrin. The active ingredient may, for example, substitute for the function of transferrin. The active ingredient can be less expensive than transferrin. Also, while transferrin is a protein and requires refrigerated storage, the active ingredient may be storable at room temperature. Therefore, the use of the active ingredient can be advantageous in terms of economy and/or storage stability as compared with the use of transferrin.

In one embodiment, a product may be produced by culturing animal cells. Examples of the product include cultured meat and a target substance. The target substance is not particularly limited as long as it can be produced by animal cells. Examples of the target substance include proteins and viruses. A particular example of the product is cultured meat. When a product is produced by culturing animal cells, the use of the active ingredient may improve the production of the product. That is, by culturing animal cells in the presence of the active ingredient, the production of the product may be improved as compared with the case where animal cells are cultured in the absence of the active ingredient. For example, by improving the growth and/or promoting the differentiation of animal cells by using the active ingredient, the production of the product may be improved. That is, one embodiment of the growth-improving effect or the differentiation-promoting effect may be an effect of improving the production of the product. The effect of improving the production of the product may be obtained, for example, when animal cells are cultured under conditions where serum is substantially absent (that is, when animal cells are cultured in a medium substantially free of serum). The effect of improving the production of the product may be obtained, for example, when animal cells are cultured under conditions where animal-derived albumin is substantially absent (that is, when animal cells are cultured in a medium substantially free of animal-derived albumin). The effect of improving the production of the product may be obtained, for example, when animal cells are cultured under conditions where serum and animal-derived albumin are substantially absent (that is, when animal cells are cultured in a medium substantially free of serum and animal-derived albumin). Improvement in production includes an improvement in the production amount and an improvement in the production rate.

An "iron ionophore" may mean a substance that has a function of increasing the permeability of iron ions across a biological membrane. Specifically, an "iron ionophore" may mean, for example, a substance that binds to an iron ion and passes through a biological membrane. Examples of the iron ionophore include hinokitiol (also referred to as "β-thujaplicin"), α-thujaplicin, and γ-thujaplicin. A particular example of the iron ionophore is hinokitiol. As the iron ionophore, one type of substance may be used, or two or more types of substances may be used in combination.

When the active ingredient can form a salt, the active ingredient may be used as a free form, as a salt, or as a combination thereof. That is, the term "active ingredient", unless otherwise specified, may mean the active ingredient in its free form, a salt thereof, or a combination thereof. The "free form" means a form that has not formed a salt. Also, when the active ingredient can form a hydrate, the active ingredient may be used as a non-hydrate, as a hydrate, or as a combination thereof. That is, the term "active ingredient" (e.g., "active ingredient in its free form" or "salt of the active ingredient"), unless otherwise specified, may include non-hydrates and hydrates. The active ingredient may take any form, such as an ion, at the time of use.

The salt is not particularly limited as long as it is acceptable for the purposes of the present invention. For example, when producing an edible product by culturing animal cells, an orally acceptable salt may be selected. For example, specific examples of salts for acidic groups such as a carboxyl group include ammonium salts; salts with alkali metals such as sodium and potassium; salts with alkaline earth metals such as calcium and magnesium; aluminum salts; zinc salts; salts with organic amines such as triethylamine, ethanolamine, morpholine, pyrrolidine, piperidine, piperazine, and dicyclohexylamine; and salts with basic amino acids such as arginine and lysine. Furthermore, for example, specific examples of salts for basic groups such as an amino group include salts with inorganic acids such as hydrochloric acid, sulfuric acid, phosphoric acid, nitric acid, and hydrobromic acid; salts with organic carboxylic acids such as acetic acid, citric acid, benzoic acid, maleic acid, fumaric acid, tartaric acid, succinic acid, tannic acid, butyric acid, hybenzic acid, pamoic acid, enanthic acid, decanoic acid, theoclic acid, salicylic acid, lactic acid, oxalic acid, mandelic acid, malic acid, methylmalonic acid, and adipic acid; and salts with organic sulfonic acids such as methanesulfonic acid, benzenesulfonic acid, and p-toluenesulfonic acid. For example, the salt of succinic acid is particularly a sodium salt. As the salt, one type of salt may be used, or two or more types of salts may be used in combination.

As the active ingredient, a commercially available product may be used, or one that has been appropriately manufactured and obtained may be used. The method for manufacturing the active ingredient is not particularly limited. The active ingredient can be manufactured, for example, by chemical synthesis, enzymatic reaction, a fermentation method, an extraction method, or a combination thereof. The active ingredient may or may not be purified to a desired degree. That is, as the active ingredient, a purified product may be used, or a material containing the active ingredient may be used. As the active ingredient, for example, a material with a content of the active ingredient of 1% (w/w) or more, 5% (w/w) or more, 10% (w/w) or more, 30% (w/w) or more, 50% (w/w) or more, 70% (w/w) or more, 90% (w/w) or more, or 95% (w/w) or more may be used.

The amount of the active ingredient (e.g., content (concentration) or amount used) shall, when using a material containing the active ingredient, be calculated based on the amount of the active ingredient itself in said material. Furthermore, the amount of the active ingredient (e.g., content (concentration) or amount used) shall, when the active ingredient forms a salt or hydrate, be calculated based on a value obtained by converting the mass of the salt or hydrate to the mass of the equimolar non-hydrated free form.

### <2> Composition of the Present Invention

The composition of the present invention is a composition containing an active ingredient (i.e., an iron ionophore).

The composition of the present invention can be used, for example, for culturing animal cells. That is, the composition of the present invention may be a composition for animal cell culture. The composition of the present invention can be used for culturing animal cells, for example, in the manner described in the method of the present invention described later.

The composition of the present invention (specifically, a composition for animal cell culture) may be, for example, a medium. Examples of the medium include a basal medium, a fed-batch medium (feed medium), and a perfusion medium. The "basal medium" may mean the medium at the start of culturing. The basal medium is also referred to as an "initial medium". The "fed-batch medium" may mean a medium that is supplied to the culture system after the start of culturing in fed-batch culture. The "perfusion medium" may mean a medium that is supplied to the culture system after the start of culturing in continuous culture (which is not limited to perfusion culture).

The composition of the present invention (specifically, a composition for animal cell culture) may be, for example, a medium supplement. A "medium supplement" may mean a composition that is used by being added to a medium. Examples of the medium to which the medium supplement is added include a basal medium, a fed-batch medium, and a perfusion medium.

By using the composition of the present invention, the growth of animal cells may be improved, that is, an effect of improving the growth of animal cells (a growth-improving effect) may be obtained. For example, a growth-improving effect may be obtained by culturing animal cells in the composition of the present invention that is a medium. Furthermore, for example, a growth-improving effect may be obtained by culturing animal cells in a medium to which the composition of the present invention, which is a medium supplement, has been added. In other words, the composition of the present invention may have a function of improving the growth of animal cells (a growth-improving function). Therefore, the composition of the present invention may be used for improving the growth of animal cells. That is, the composition of the present invention may be, for example, a composition for improving the growth of animal cells.

By using the composition of the present invention, the differentiation of animal cells may be promoted, that is, an effect of promoting the differentiation of animal cells (a differentiation-promoting effect) may be obtained. For example, a differentiation-promoting effect may be obtained by culturing animal cells in the composition of the present invention that is a medium. Furthermore, for example, a differentiation-promoting effect may be obtained by culturing animal cells in a medium to which the composition of the present invention, which is a medium supplement, has been added. In other words, the composition of the present invention may have a function of promoting the differentiation of animal cells (a differentiation-promoting function). Therefore, the composition of the present invention may be used for promoting the differentiation of animal cells. That is, the composition of the present invention may be, for example, a composition for promoting the differentiation of animal cells.

By using the composition of the present invention, at least a growth-improving effect may be obtained.

By using the composition of the present invention, a growth-improving effect and a differentiation-promoting effect may be obtained. That is, the composition of the present invention may be, for example, a composition for improving the growth and promoting the differentiation of animal cells.

In one embodiment, a product (e.g., cultured meat or a target substance) may be produced by culturing animal cells. When a product is produced by culturing animal cells, the use of the composition of the present invention may improve the production of the product. For example, by improving the growth and/or promoting the differentiation of animal cells by using the composition of the present invention, the production of the product may be improved. That is, one embodiment of the composition of the present invention (specifically, a composition for animal cell culture) may be a composition for improving the production of a product.

The composition of the present invention may consist of the active ingredient, or it may contain components other than the active ingredient. Components other than the active ingredient are also referred to as "additional components". That is, the active ingredient may be used as the composition of the present invention as it is, or in combination with additional components. As the additional components, one type of component may be used, or two or more types of components may be used in combination.

That is, the present invention provides a method for producing the composition of the present invention, which includes using the active ingredient. The mode of use of the active ingredient in the production of the composition of the present invention is not particularly limited as long as a composition containing the active ingredient is obtained. The use of the active ingredient in the production of the composition of the present invention includes incorporating the active ingredient into the composition. "Incorporating the active ingredient into the composition" may mean causing the composition to contain the active ingredient. "Incorporating the active ingredient into the composition" may specifically mean combining the active ingredient with additional components.

The additional components are not particularly limited as long as the object of the present invention can be achieved (e.g., a growth-improving effect or a differentiation-promoting effect is obtained). The additional components can be appropriately selected, for example, according to various conditions such as the type of animal cell and the mode of use of the composition of the present invention. Examples of additional components include medium components. Medium components will be described later. For example, at least when the composition of the present invention is a medium, the composition of the present invention may contain medium components. A specific example of an additional component is iron. That is, the composition of the present invention may, for example, contain iron.

The composition of the present invention may, for example, be appropriately formulated. In formulation, additives may be used as appropriate. That is, examples of additional components also include additives used in formulation. Examples of additives include excipients, binders, disintegrants, lubricants, stabilizers, flavoring and odor-masking agents, diluents, and surfactants. The additives can be appropriately selected, for example, according to various conditions such as the shape of the composition of the present invention.

The shape of the composition of the present invention is not particularly limited. The composition of the present invention may be in any shape, for example, powder, flakes, tablets, paste, or liquid.

The content and content ratio of each component (i.e., the active ingredient and optional additional components) in the composition of the present invention are not particularly limited as long as the object of the present invention can be achieved (e.g., a growth-improving effect or a differentiation-promoting effect is obtained). That is, for example, the content of the active ingredient in the composition of the present invention may be an effective amount (e.g., an amount that provides a growth-improving effect or a differentiation-promoting effect). The content and content ratio of each component in the composition of the present invention can be appropriately set according to various conditions such as the type of animal cell and the mode of use of the composition of the present invention.

The content of the active ingredient in the composition of the present invention is more than 0% (w/w) and 100% (w/w) or less. The content of the active ingredient in the composition of the present invention may be, for example, 1 ppb (w/w) or more, 10 ppb (w/w) or more, 100 ppb (w/w) or more, 1 ppm (w/w) or more, 10 ppm (w/w) or more, 100 ppm (w/w) or more, 1000 ppm (w/w) or more, 1% (w/w) or more, 2% (w/w) or more, 5% (w/w) or more, or 10% (w/w) or more, and may be 100% (w/w) or less, less than 100% (w/w), 99.9% (w/w) or less, 90% (w/w) or less, 50% (w/w) or less, 20% (w/w) or less, 10% (w/w) or less, 5% (w/w) or less, 2% (w/w) or less, 1% (w/w) or less, 1000 ppm (w/w) or less, 100 ppm (w/w) or less, 10 ppm (w/w) or less, or 1 ppm (w/w) or less, or any non-contradictory combination thereof. Specifically, the content of the active ingredient in the composition of the present invention may be, for example, 1 ppb (w/w) to 1 ppm (w/w), 1 ppm (w/w) to 10 ppm (w/w), 10 ppm (w/w) to 100 ppm (w/w), 100 ppm (w/w) to 1000 ppm (w/w), 1000 ppm (w/w) to 1% (w/w), 1% (w/w) to 10% (w/w), or 10% (w/w) to 20% (w/w). Specifically, the content of the active ingredient in the composition of the present invention may be, for example, 1 ppb (w/w) to 10% (w/w), 1 ppb (w/w) to 1% (w/w), or 1 ppb (w/w) to 1000 ppm (w/w). When the composition of the present invention contains two or more active ingredients, the content of these two or more active ingredients in the composition of the present invention may be set, independently or in total, within the range of the content of the active ingredient in the composition of the present invention exemplified above (provided that the total content of these two or more active ingredients in the composition of the present invention is 100% (w/w) or less). When the composition of the present invention contains two or more active ingredients, "the content of the active ingredient in the composition of the present invention" shall, unless otherwise specified, mean the total content of these two or more active ingredients in the composition of the present invention.

In one embodiment, the composition of the present invention may be substantially free of serum. "The composition of the present invention being substantially free of serum" may mean that the serum content in the composition of the present invention is 1% (w/w) or less, 0.1 % (w/w) or less, 0.01% (w/w) or less, or 0.001% (w/w) or less, and may also include the case where the serum content in the composition of the present invention is 0 (zero) (i.e., the composition of the present invention does not contain serum).

In one embodiment, the composition of the present invention may be substantially free of animal-derived albumin. "The composition of the present invention being substantially free of animal-derived albumin" may mean that the content of animal-derived albumin in the composition of the present invention is 1% (w/w) or less, 0.1% (w/w) or less, 0.01% (w/w) or less, or 0.001% (w/w) or less, and may also include the case where the content of animal-derived albumin in the composition of the present invention is 0 (zero) (i.e., the composition of the present invention does not contain animal-derived albumin).

In one embodiment, the composition of the present invention may be substantially free of transferrin. "The composition of the present invention being substantially free of transferrin" may mean that the content of transferrin in the composition of the present invention is 0.001% (w/w) or less, 0.0001% (w/w) or less, 0.00001% (w/w) or less, or 0.000001% (w/w) or less, and may also include the case where the content of transferrin in the composition of the present invention is 0 (zero) (i.e., the composition of the present invention does not contain transferrin).

The content of each component (i.e., the active ingredient and optional additional components) in the composition of the present invention can be set, for example, so as to obtain the concentration of each component in the medium in the method of the present invention described later.

When the composition of the present invention contains two or more components, these components may be mixed with each other and contained in the composition of the present invention, or they may be contained in the composition of the present invention separately, either individually or in any combination. For example, the composition of the present invention may be provided as a set of a package of the active ingredient and a package of the additional components. In such a case, the components included in the set can be used in combination as appropriate at the time of use.

### <3> Method of the Present Invention

The method of the present invention is a method for culturing animal cells, which includes a step of culturing animal cells in the presence of an active ingredient (i.e., an iron ionophore). This step is also referred to as the "animal cell culturing step".

By using the active ingredient, specifically by culturing animal cells in the presence of the active ingredient, the growth of animal cells may be improved, that is, an effect of improving the growth of animal cells (a growth-improving effect) may be obtained. That is, one embodiment of the method of the present invention (specifically, a method for culturing animal cells) may be, for example, a method for improving the growth of animal cells.

By using the active ingredient, specifically by culturing animal cells in the presence of the active ingredient, the differentiation of animal cells may be promoted, that is, an effect of promoting the differentiation of animal cells (a differentiation-promoting effect) may be obtained. That is, one embodiment of the method of the present invention (specifically, a method for culturing animal cells) may be, for example, a method for promoting the differentiation of animal cells.

By using the active ingredient, specifically by culturing animal cells in the presence of the active ingredient, at least a growth-improving effect may be obtained.

By using the active ingredient, specifically by culturing animal cells in the presence of the active ingredient, a growth-improving effect and a differentiation-promoting effect may be obtained. That is, one embodiment of the method of the present invention (specifically, a method for culturing animal cells) may be, for example, a method for improving the growth and promoting the differentiation of animal cells.

The purpose of culturing animal cells is not particularly limited.

In one embodiment, a product (e.g., cultured meat or a target substance) may be produced by culturing animal cells. That is, when animal cells can form cultured meat through culturing, cultured meat can be produced by culturing said cells. The cultured meat may be mainly composed of muscle tissue. Therefore, "animal cells can form cultured meat through culturing" may mean, for example, that animal cells can differentiate and form muscle tissue through culturing, and specifically, it may mean that animal cells can differentiate into myotubes and form muscle tissue through culturing. Furthermore, when animal cells have the ability to produce a target substance, the target substance can be produced by culturing said cells. That is, one embodiment of the method of the present invention (specifically, a method for culturing animal cells) may be a method for producing a product, including a step of culturing animal cells in the presence of an active ingredient. The animal cell culturing step may also specifically be a step of producing a product by culturing animal cells in the presence of an active ingredient. In the method for producing cultured meat, the cultured animal cells may form cultured meat. In the method for producing a target substance, the cultured animal cells may produce the target substance. In other words, when animal cells can produce a target substance through culturing, the target substance can be produced by culturing said cells. Examples of the target substance include proteins and viruses. In cases where the target substance includes a virus, the method for producing the target substance may further include a step of causing the animal cells to produce the virus. When producing a product by culturing animal cells, the use of the active ingredient may improve the production of the product. For example, by improving the growth and/or promoting the differentiation of animal cells by using the active ingredient, the production of the product may be improved. That is, one embodiment of the method of the present invention (specifically, a method for culturing animal cells) may be a method for improving the production of a product.

"Animal cell" means a cell of an animal. The animal cell is not particularly limited. The animal cell can be appropriately selected, for example, according to various conditions such as the purpose of culturing the animal cells. For example, when producing cultured meat by culturing animal cells, the animal cell is not particularly limited as long as it can form cultured meat through culturing. "Animal" may mean an organism classified in the kingdom Animalia. Examples of animals include vertebrates and aquatic organisms. Examples of vertebrates include mammals, birds, reptiles, amphibians, and fish. Vertebrates particularly include mammals, birds, and fish. Vertebrates particularly include mammals. Examples of mammals include primates such as humans, monkeys, and chimpanzees; rodents such as hamsters, mice, rats, and guinea pigs; other terrestrial mammals such as cows, pigs, sheep, goats, rabbits, horses, deer, water buffalos, reindeer, donkeys, camels, dogs, and cats; and aquatic mammals such as whales, dolphins, reindeer, and walruses. Examples of birds include chickens, turkeys, ducks, geese, guinea fowls, quails, and ostriches. Examples of fish include eels, tuna, longtooth groupers, sea breams, salmon, cod, and pufferfish. In addition to aquatic mammals and fish, examples of aquatic organisms include crustaceans such as shrimps and crabs, shellfish such as scallops and oysters, and other aquatic organisms such as squid and octopus. Aquatic organisms particularly include fish and crustaceans. Animals suitable for the production of cultured meat include animals for meat production. Animals for meat production include those among the animals exemplified above whose meat can be used for food. Animals for meat production (especially mammals) particularly include cows, pigs, sheep, goats, and rabbits. Animals for meat production (especially mammals) more particularly include cows and pigs. Animals for meat production (especially mammals) even more particularly include cows. Animals for meat production (especially birds) particularly include chickens, turkeys, ducks, geese, guinea fowls, quails, and ostriches. Animals for meat production (especially birds) more particularly include chickens. Animals for meat production (especially aquatic organisms) particularly include the fish and crustaceans exemplified above. Animals for meat production (especially aquatic organisms) more particularly include eels, tuna, and shrimps. The tissue or cell from which the animal cell is derived is not particularly limited. Examples of the tissue or cell from which the animal cell is derived include ovary, kidney, adrenal gland, tongue epithelium, olfactory epithelium, pineal body, thyroid, melanocyte, skin, spleen, liver, lung, pancreas, uterus, stomach, colon, small intestine, large intestine, bladder, prostate, testis, thymus, muscle, connective tissue, bone, cartilage, vascular tissue, blood (including umbilical cord blood), bone marrow, heart, eye, brain, and nervous tissue. The animal cells may or may not be differentiated. Specific examples of animal cells include germ cells, somatic cells, stem cells, and progenitor cells. Examples of germ cells include sperm and eggs. Examples of somatic cells include myoblasts, fibroblasts, bone marrow cells, B lymphocytes, T lymphocytes, neutrophils, red blood cells, platelets, macrophages, monocytes, bone cells, pericytes, dendritic cells, fat cells, mesenchymal cells, epithelial cells, epidermal cells (e.g., keratinocytes, corneocytes, etc.), endothelial cells, vascular endothelial cells, hepatocytes, chondrocytes, cumulus cells, nerve cells, glial cells, oligodendrocytes, microglia, astrocytes, heart cells, esophageal cells, muscle cells (e.g., smooth muscle cells, skeletal muscle cells), pancreatic beta cells, melanocytes, and mononuclear cells. Examples of stem cells include adult stem cells such as hematopoietic stem cells, satellite cells (muscle stem cells), neural stem cells, mesenchymal stem cells, mammary gland stem cells, olfactory mucosal stem cells, neural crest stem cells, liver stem cells, pancreatic stem cells, germline stem cells, intestinal stem cells, and hair follicle stem cells; pluripotent stem cells such as embryonic stem cells (ES cells), embryonic carcinoma cells, embryonic germ stem cells, and induced pluripotent stem cells (iPS cells); and cancer stem cells. Examples of progenitor cells include satellite cells, pancreatic progenitor cells, vascular progenitor cells, vascular endothelial progenitor cells, and hematopoietic progenitor cells (such as CD34-positive cells derived from umbilical cord blood). Animal cells suitable for the production of cultured meat include stem cells such as satellite cells (muscle stem cells), mesenchymal stem cells, embryonic stem cells (ES cells), and induced pluripotent stem cells (iPS cells), and myoblasts. Animal cells suitable for the production of cultured meat particularly include satellite cells (muscle stem cells) and myoblasts. Myoblasts may be classified as stem cells.

"An animal cell is a cell that satisfies a certain condition" means that it is sufficient for the animal cell to satisfy said condition during at least a part of the culture period, and it is not required for the animal cell to satisfy said condition for the entire culture period. That is, "an animal cell is a cell that satisfies a certain condition" is not limited to cases where the animal cell satisfies said condition at the start of culturing (i.e., starting the culture using an animal cell that satisfies said condition), but may also include cases where the animal cell comes to satisfy said condition after the start of culturing. For example, "the animal cell is a myoblast" is not limited to cases where the culture is started using animal cells that are myoblasts, but may also include cases where the animal cells differentiate into myoblasts after the start of culturing.

The culturing of animal cells is carried out in the presence of the active ingredient. "The culturing of animal cells is carried out in the presence of the active ingredient" may mean, for example, culturing the animal cells in a medium containing the active ingredient. "The culturing of animal cells is carried out in the presence of the active ingredient" may also mean, for example, that the active ingredient is supplied to the culture system during the culturing of the animal cells.

The active ingredient may be used for culturing animal cells, for example, in the form of the composition of the present invention. The composition of the present invention may be used for culturing animal cells, for example, as a medium. That is, for example, when the composition of the present invention is a medium, the animal cells may be cultured in the composition of the present invention (i.e., the medium). That is, "culturing the animal cells in a medium containing the active ingredient" may include culturing the animal cells in the composition of the present invention that is a medium. The composition of the present invention may also be used for culturing animal cells, for example, as a medium supplement. That is, for example, when the composition of the present invention is a medium supplement, the composition of the present invention (i.e., the medium supplement) may be added to a medium, and the animal cells may be cultured in the medium to which the composition of the present invention has been added. That is, "culturing the animal cells in a medium containing the active ingredient" may include culturing the animal cells in a medium to which the composition of the present invention, which is a medium supplement, has been added. Also, for example, the composition of the present invention or a medium to which it has been added may be supplied to the culture system during the culturing of the animal cells. That is, "the active ingredient is supplied to the culture system during the culturing of the animal cells" may include that the composition of the present invention or a medium to which it has been added is supplied to the culture system during the culturing of the animal cells.

The medium composition and culture conditions are not particularly limited as long as the object of culturing the animal cells can be achieved, except that the culturing of animal cells is carried out in the presence of the active ingredient. The medium composition and culture conditions can be appropriately set, for example, according to various conditions such as the type of animal cell and the purpose of culturing the animal cells. For example, when producing cultured meat by culturing animal cells, the medium composition and culture conditions are not particularly limited as long as cultured meat can be formed by culturing the animal cells. The culturing of animal cells, except for being carried out in the presence of the active ingredient, can be performed, for example, using a normal medium and normal conditions used for culturing animal cells as they are, or by modifying them as appropriate. When producing cultured meat by culturing animal cells, the culturing of animal cells, except for being carried out in the presence of the active ingredient, can be performed, for example, using a normal medium and normal conditions used for the formation of muscle tissue by differentiation of animal cells (e.g., for the production of cultured meat) as they are, or by modifying them as appropriate.

The culturing can be carried out, for example, using a liquid medium. The culturing can be carried out by batch culture, fed-batch culture, continuous culture, or a combination thereof. Examples of continuous culture include perfusion culture and chemostat culture. The medium at the start of culturing is also referred to as an "initial medium" or "basal medium". The medium supplied to the culture system (e.g., the initial medium) in fed-batch culture is also referred to as a "fed-batch medium (feed medium)". The medium supplied to the culture system (e.g., the initial medium) in continuous culture (which is not limited to perfusion culture) is also referred to as a "perfusion medium". Furthermore, supplying a feed medium or a perfusion medium to the culture system in fed-batch culture or continuous culture is also simply referred to as "medium supply". Medium supply may be carried out throughout the entire culture period, or only during a part of the culture period. Medium supply may be carried out continuously or intermittently. During culturing (especially continuous culture such as perfusion culture), withdrawal of the culture medium may be carried out. The withdrawal of the culture medium may be carried out throughout the entire culture period, or only during a part of the culture period. The withdrawal of the culture medium may be carried out continuously or intermittently. The withdrawal of the culture medium and the medium supply may or may not be carried out simultaneously. The culture vessel may be coated with a cell adhesion molecule such as fibronectin, for example. The culturing may be carried out three-dimensionally, for example, using a scaffold material. When producing cultured meat by culturing animal cells, the culturing may be carried out three-dimensionally, for example, using a scaffold material shaped to match the shape of the cultured meat to be formed.

The medium used for culturing can be selected independently, for example, for the basal medium, the fed-batch medium, and the perfusion medium.

The medium used for culturing may be a commercially available medium or a medium prepared as appropriate.

Examples of the medium used for culturing include a medium containing components essential for culturing animal cells (e.g., a carbon source, a nitrogen source, inorganic salts, etc.).

Specific examples of the medium used for culturing include Dulbecco's Modified Eagle's Medium (DMEM), Ham's Nutrient Mixture F12, DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB131 medium, William's Medium E, and Fischer's Medium.

Specific examples of the medium used for culturing (which may be a medium used particularly for culturing stem cells (especially pluripotent stem cells)) include STEMPRO^{®} hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO^{™} Serum-Free Medium for hES cells (Millipore), PluriSTEM^{™} Human ES/iPS Medium (EMD Millipore), NutriStem^{®} hESC XF medium (Biological Industries Israel Beit-Haemek), NutriStem^{™} XF/FF Culture Medium (Stemgent), AF NutriStem^{®} hESC XF medium (Biological Industries Israel Beit-Haemek), S-medium (DS Pharma Biomedical Co., Ltd.), StemFit^{®} AK03N medium (Ajinomoto Co., Inc.), hESF9 medium, hESF-FX medium, CDM medium, DEF-CS 500 Xeno-Free 3D Spheroid Culture Medium (Cellartis), StemFlex medium (Thermo Fisher Scientific), and the like.

Other commercially available media include media for animal cell culture such as CELLiST Basal Media BASAL3, BASAL10 (Ajinomoto Co., Inc.), Opti-MEM (Thermo Fisher Scientific), RPMI 1640 (Thermo Fisher Scientific), CD293 (Thermo Fisher Scientific), CHO-S-SFMII (Thermo Fisher Scientific), CHO-SF (Sigma-Aldrich), EX-CELL CD CHO (Sigma-Aldrich), EX-CELL^{™}302 (Sigma-Aldrich), IS CHO-CD (Irvine Scientific), and IS CHO-CDXP (Irvine Scientific).

The media exemplified above may be used for culturing, for example, by adding the active ingredient.

The medium used for culturing may be, for example, the composition of the present invention (specifically, the composition of the present invention that is a medium). That is, when the composition of the present invention is a medium, the composition of the present invention may be used for culturing as it is, or after being prepared as a liquid medium of a desired composition as appropriate. For example, the composition of the present invention may be diluted with an aqueous medium such as water or an aqueous buffer to be prepared as a liquid medium and used for culturing.

The medium used for culturing may be, for example, a medium to which the composition of the present invention (specifically, the composition of the present invention that is a medium supplement) has been added. The medium to which the composition of the present invention is added may be a commercially available medium or a medium prepared as appropriate.

The medium may contain various medium components. Examples of medium components include carbon sources, amino acid sources, peptides, proteins, vitamins, fatty acids, lipids, organic acids, nucleic acids, amines, antioxidants, inorganic components, pH buffering agents, growth factors, cytokines, hormones, cell adhesion factors, extracellular matrix components, serum, antibiotics, and gene expression inducers. Any of these medium components may be, for example, essential or effective for the survival or proliferation of animal cells. Any of these medium components may, for example, be pre-contained in the media as exemplified above, or may be added to the media as exemplified above.

Examples of the carbon source include sugars such as glucose, fructose, sucrose, and maltose.

Examples of the amino acid source include amino acids. Peptides and proteins can both be examples of amino acid sources. Examples of amino acids include glycine, alanine, valine, leucine, isoleucine, cysteine, methionine, phenylalanine, tyrosine, tryptophan, histidine, lysine, arginine, serine, threonine, aspartic acid, glutamic acid, asparagine, glutamine, proline, and ornithine. The amino acids may be, for example, in the L-form.

Examples of peptides include dipeptides and tripeptides. Specific examples of peptides include glycyl-glycyl-glycine and soy peptide. The description concerning amino acids can be applied mutatis mutandis to the amino acids constituting the peptides.

### Examples of proteins include albumin and transferrin.

Examples of vitamins include vitamin A, vitamin B1, vitamin B2, vitamin B3, vitamin B5, vitamin B6, vitamin B7, vitamin B9, vitamin B12, vitamin C, vitamin D, vitamin E, and vitamin K, and their precursors.

Examples of fatty acids include oleic acid, arachidonic acid, and linoleic acid.

An example of a lipid is cholesterol.

An example of an organic acid is pyruvic acid (such as sodium pyruvate).

Examples of nucleic acids include hypoxanthine and thymidine.

An example of an amine is a polyamine such as putrescine.

An example of an antioxidant is lipoic acid.

Examples of inorganic components include sodium, potassium, calcium, magnesium, phosphorus, and various trace elements (e.g., Co, Cu, F, Fe, Mn, Mo, Ni, Se, Si, Ni, Bi, V, and Zn). Specific examples of inorganic components include inorganic salts such as sodium chloride, potassium chloride, calcium chloride, magnesium sulfate, and sodium dihydrogen phosphate. A particular example of an inorganic component is iron. The iron may be, for example, ferrous iron, ferric iron, or a mixture thereof. An example of iron is an iron salt. Examples of iron salts include iron sulfate (e.g., iron(II) sulfate), iron nitrate (e.g., iron(III) nitrate and iron(II) nitrate), iron chloride (e.g., iron(III) chloride and iron(II) chloride), iron sulfide (e.g., iron(III) sulfide and iron(II) sulfide), and ferric ammonium citrate (e.g., ferric(III) ammonium citrate). The iron (e.g., an iron salt) may be, for example, a hydrate. As the iron, one type of component may be used, or two or more types of components may be used in combination.

Examples of pH buffering agents include sodium bicarbonate, phosphate, N,N-bis(2-hydroxyethyl)-2-aminoethanesulfonic acid (BES), 4-(2-hydroxyethyl)-1-piperazineethanesulfonic acid (HEPES), and N-[tris(hydroxymethyl)methyl]glycine (Tricine).

Examples of growth factors include fibroblast growth factor (FGF), hepatocyte growth factor (HGF), epidermal growth factor (EGF), transforming growth factor (TGF)-α, transforming growth factor (TGF)-β, vascular endothelial growth factor (VEGF), activin A, and insulin-like growth factor-1 (IGF-1). An example of FGF is basic fibroblast growth factor (bFGF). A particular example of a growth factor is bFGF. That is, as a medium component, at least bFGF may be used. That is, the medium may contain at least bFGF. An example of bFGF is bovine basic fibroblast growth factor (bbFGF). "Bovine basic fibroblast growth factor (bbFGF)" may mean bFGF that is bovine-derived. "Bovine" in relation to bbFGF may mean an organism of the genus Bos. An example of an organism of the genus Bos is Bos taurus. bbFGF is not limited to bFGF found in organisms of the genus Bos, and may be a variant thereof.

An example of a cytokine is an interleukin.

Examples of hormones include dexamethasone, hydrocortisone, estradiol, progesterone, glucagon, and insulin.

Examples of cell adhesion factors or extracellular matrix components include Type I collagen, Type II collagen, fibronectin, laminin, poly-L-lysine, and poly-D-lysine.

Examples of antibiotics include amphotericin B, kanamycin, gentamicin, streptomycin, and penicillin.

In one embodiment, the medium may be substantially free of serum. The medium may be substantially free of serum, for example, for the entire duration of the culture. "A medium being substantially free of serum" may mean that the serum content in the medium is 1% (w/w) or less, 0.1% (w/w) or less, 0.01% (w/w) or less, or 0.001% (w/w) or less, and may also include cases where the serum content in the medium is 0 (zero) (i.e., the medium does not contain serum).

In one embodiment, the medium may be substantially free of animal-derived albumin. Examples of the animal from which the albumin is derived include the animals exemplified for the animal cells. The medium may be substantially free of animal-derived albumin, for example, for the entire duration of the culture. "A medium being substantially free of animal-derived albumin" may mean that the content of animal-derived albumin in the medium is 1% (w/w) or less, 0.1 % (w/w) or less, 0.01% (w/w) or less, or 0.001% (w/w) or less, and may also include cases where the content of animal-derived albumin in the medium is 0 (zero) (i.e., the medium does not contain animal-derived albumin).

Any of the various components, such as the active ingredient, may be contained in the initial medium, the fed-batch medium, the perfusion medium, or a combination thereof. That is, during the culturing process, various components such as the active ingredient may be supplied to the medium, either alone or in any combination. Any of these components may be supplied once or multiple times, or may be supplied continuously. The composition (e.g., the types and/or concentrations of the components contained) of the initial medium, the fed-batch medium, and the perfusion medium may or may not be the same. That is, the types of components contained in the initial medium may or may not be the same as the types of components contained in the fed-batch or perfusion medium. Furthermore, the concentration of each component contained in the initial medium may or may not be the same as the concentration of each component contained in the fed-batch or perfusion medium. For example, when a fed-batch medium is used in perfusion culture, the composition of the initial medium and the fed-batch medium may be the same. Also, two or more fed-batch or perfusion media with different compositions (e.g., types and/or concentrations of components contained) may be used. For example, when multiple supplies of the fed-batch or perfusion medium are performed intermittently, the composition of the fed-batch or perfusion medium may or may not be the same each time. Also, various components such as the active ingredient may each be supplied to the medium in a form not contained in the fed-batch or perfusion medium, such as a powder.

The seeding amount of animal cells at the start of culturing may be, for example, in terms of viable cell count, 1×10² cells/mL or more, 1×10⁸ cells/mL or more, 1×10⁴ cells/mL or more, 1×10⁵ cells/mL or more, 1×10⁸ cells/mL or more, or 1×10⁷ cells/mL or more, and may be 1×10⁸ cells/mL or less, 1×10⁷ cells/mL or less, 1×10⁸ cells/mL or less, 1×10⁵ cells/mL or less, 1×10⁴ cells/mL or less, or 1×10⁸ cells/mL or less, or any non-contradictory combination thereof. Specifically, the seeding amount of animal cells at the start of culturing may be, for example, in terms of viable cell count, 1×10² to 1×10⁸ cells/mL, 1×10⁸ to 1×10⁴ cells/mL, 1×10⁴ to 1×10⁵ cells/mL, 1×10⁵ to 1×10⁸ cells/mL, 1×10⁶ to 1×10⁷ cells/mL, or 1×10⁷ to 1×10⁸ cells/mL. Specifically, the seeding amount of animal cells at the start of culturing may be, for example, in terms of viable cell count, 1×10² to 1×10⁸ cells/mL, 1×10⁸ to 1×10⁷ cells/mL, or 1×10⁸ to 1×10⁸ cells/mL. The viable cell count can be measured, for example, using a Vi-CELL^{™} XR Cell Viability Analyzer (Beckman Coulter, Inc.).

The culturing may be carried out, for example, in a CO₂-containing atmosphere, such as 5-15% CO₂. The pH of the medium may be, for example, around neutral. "Around neutral" may mean, for example, a pH of 6-8, a pH of 6.5-7.5, or a pH of 6.8-7.2. During culturing, the pH of the medium can be adjusted as necessary. The pH of the medium can be adjusted using various alkaline or acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 30-38°C. The culture period may be, for example, 0.5 days or more, 1 day or more, 2 days or more, 3 days or more, 4 days or more, 5 days or more, 6 days or more, 7 days or more, 8 days or more, 9 days or more, 10 days or more, 12 days or more, 15 days or more, or 20 days or more, and may be 60 days or less, 50 days or less, 40 days or less, 30 days or less, 25 days or less, 20 days or less, 15 days or less, 12 days or less, 10 days or less, 9 days or less, 8 days or less, or 7 days or less, or any non-contradictory combination thereof. Specifically, the culture period may be, for example, 1-60 days, 3-25 days, or 5-20 days. The culturing may be continued, for example, until the product is produced to a desired extent.

The amount used (e.g., concentration in the medium) and the ratio used (e.g., concentration ratio in the medium) of each component (i.e., the active ingredient and optional additional components) in the method of the present invention are not particularly limited as long as the object of the present invention can be achieved (e.g., a growth-improving effect or a differentiation-promoting effect is obtained). That is, for example, the amount of the active ingredient used in the method of the present invention may be an effective amount (e.g., an amount that provides a growth-improving effect or a differentiation-promoting effect). Specifically, for example, the concentration of the active ingredient in the medium may be an effective concentration (e.g., a concentration that provides a growth-improving effect or a differentiation-promoting effect). The amount used and the ratio used of each component in the method of the present invention can be appropriately set according to various conditions such as the type of animal cell and the mode of use of the composition of the present invention.

The concentration of the active ingredient in the medium may be, for example, 1 nM or more, 3 nM or more, 5 nM or more, 10 nM or more, 30 nM or more, 50 nM or more, 100 nM or more, 300 nM or more, 400 nM or more, 500 nM or more, 600 nM or more, 700 nM or more, 1 µM or more, 1.5 µM or more, 2 µM or more, 2.5 µM or more, 3 µM or more, 5 µM or more, 7 µM or more, 10 µM or more, 15 µM or more, 20 µM or more, 25 µM or more, 30 µM or more, 40 µM or more, 50 µM or more, or 70 µM or more, and may be 100 µM or less, 70 µM or less, 50 µM or less, 40 µM or less, 30 µM or less, 25 µM or less, 20 µM or less, 15 µM or less, 10 µM or less, 7 µM or less, 5 µM or less, 3 µM or less, 2.5 µM or less, 2 µM or less, 1.5 µM or less, 1 µM or less, 700 nM or less, 600 nM or less, 500 nM or less, 400 nM or less, 300 nM or less, 100 nM or less, 50 nM or less, 30 nM or less, 10 nM or less, 5 nM or less, or 3 nM or less, or any non-contradictory combination thereof. Specifically, the concentration of the active ingredient in the medium may be, for example, 1 nM to 3 nM, 3 nM to 5 nM, 5 nM to 10 nM, 10 nM to 30 nM, 30 nM to 50 nM, 50 nM to 100 nM, 100 nM to 300 nM, 300 nM to 400 nM, 400 nM to 500 nM, 500 nM to 600 nM, 600 nM to 700 nM, 700 nM to 1 µM, 1 µM to 1.5 µM, 1.5 µM to 2 µM, 2 µM to 2.5 µM, 2.5 µM to 3 µM, 3 µM to 5 µM, 5 µM to 7 µM, 7 µM to 10 µM, 10 µM to 15 µM, 15 µM to 20 µM, 20 µM to 25 µM, 25 µM to 30 µM, 30 µM to 40 µM, 40 µM to 50 µM, 50 µM to 70 µM, or 70 µM to 100 µM. Specifically, the concentration of the active ingredient in the medium may be, for example, 1 nM to 100 µM, 5 nM to 30 µM, 10 nM to 10 µM, or 10 nM to 1 µM. In one embodiment, the concentration of the active ingredient in the medium may be, in particular, 2.5 µM or less, 2 µM or less, 1.5 µM or less, 1 µM or less, 700 nM or less, 600 nM or less, 500 nM or less, or 400 nM or less. For example, when at least hinokitiol is selected as the active ingredient, the concentration of hinokitiol or the total concentration of active ingredients including hinokitiol in the medium may be 2.5 µM or less, 2 µM or less, 1.5 µM or less, 1 µM or less, 700 nM or less, 600 nM or less, 500 nM or less, or 400 nM or less. In one embodiment, the concentration of the active ingredient in the medium may be, for example, specifically, 1 nM to 2.5 µM, 3 nM to 2 µM, 5 nM to 1.5 µM, or 10 nM to 1 µM. For example, when at least hinokitiol is selected as the active ingredient, the concentration of hinokitiol or the total concentration of active ingredients including hinokitiol in the medium may be 1 nM to 2.5 µM, 3 nM to 2 µM, 5 nM to 1.5 µM, or 10 nM to 1 µM. When the medium contains two or more active ingredients, the concentration of these two or more active ingredients in the medium may be set, independently or in total, within the range of the concentration of the active ingredient in the medium exemplified above. When the medium contains two or more active ingredients, "the concentration of the active ingredient in the medium" shall, unless otherwise specified, mean the total concentration of these two or more active ingredients in the medium.

The culturing of animal cells may be performed in the presence of iron. "The culturing of animal cells is performed in the presence of a certain component (e.g., iron)" may mean, for example, culturing the animal cells in a medium containing said component. "The culturing of animal cells is performed in the presence of a certain component (e.g., iron)" may also mean, for example, that said component is supplied to the culture system during the culturing of the animal cells.

When the medium contains iron, the concentration of iron in the medium may be, for example, 200 nM or more, 500 nM or more, 1 µM or more, 1.5 µM or more, 2 µM or more, 3 µM or more, 5 µM or more, 7 µM or more, 10 µM or more, 15 µM or more, 20 µM or more, 25 µM or more, 30 µM or more, 40 µM or more, 50 µM or more, 70 µM or more, 100 µM or more, 150 µM or more, or 200 µM or more, and may be 500 µM or more, 200 µM or more, 150 µM or more, 100 µM or more, 70 µM or more, 50 µM or more, 40 µM or more, 30 µM or more, 25 µM or more, 20 µM or more, 15 µM or more, 10 µM or more, 7 µM or more, 5 µM or more, 3 µM or more, 2 µM or more, 1.5 µM or more, 1 µM or more, or 500 nM or more, or any non-contradictory combination thereof. Specifically, the concentration of iron in the medium may be, for example, 200 nM to 500 nM, 500 nM to 1 µM, 1 µM to 1.5 µM, 1.5 µM to 2 µM, 2 µM to 3 µM, 3 µM to 5 µM, 5 µM to 7 µM, 7 µM to 10 µM, 10 µM to 15 µM, 15 µM to 20 µM, 20 µM to 25 µM, 25 µM to 30 µM, 30 µM to 40 µM, 40 µM to 50 µM, 50 µM to 70 µM, 70 µM to 100 µM, 100 µM to 150 µM, 150 µM to 200 µM, or 200 µM to 500 µM. Specifically, the concentration of iron in the medium may be, for example, 200 nM to 500 µM, 500 nM to 100 µM, 1 µM to 50 µM, or 1.5 µM to 30 µM. When the medium contains two or more forms of iron, the concentration of these two or more forms of iron in the medium may be set, independently or in total, within the range of the concentration of iron in the medium exemplified above. When the medium contains two or more forms of iron, "the concentration of iron in the medium" shall, unless otherwise specified, mean the total concentration of these two or more forms of iron in the medium. The molar concentration of iron is calculated based on the number of moles of iron constituting the compound. For example, in the case of 1 µM of iron(III) sulfide (Fe₂S₃), the molar concentration of iron is 2 µM.

Any of the various components, such as the active ingredient, may be contained in the medium for the entire duration of the culture, or may be contained in the medium for only a part of the culture period. That is, "culturing is carried out in a medium containing a certain component" means that it is sufficient for said component to be contained in the medium for at least a part of the culture period, and it is not required for said component to be contained in the medium for the entire duration of the culture. For example, the active ingredient and iron may coexist in the medium for at least a part of the culture period. Any of the various components, such as the active ingredient, may, for example, be contained in the medium at the start of culturing, or may be supplied to the medium after the start of culturing. Also, any of the various components, such as the active ingredient, may, for example, be contained in the medium at the start of culturing and also be further supplied to the medium after the start of culturing (e.g., after consumption of the active ingredient).

Any of the various components, such as the active ingredient, may be contained in the medium at the exemplified concentrations for the entire duration of the culture, for example, or may be contained in the medium at the exemplified concentrations for only a part of the culture period. That is, "culturing is carried out in a medium containing a certain component at a certain concentration," "a certain component is contained in the medium during culturing at a certain concentration," or "the concentration of a certain component in the medium during culturing is a certain concentration" means that it is sufficient for the concentration of said component in the medium to be within the range of said concentration for at least a part of the culture period, and it is not required for the concentration of said component in the medium to be within the range of said concentration for the entire duration of the culture. Any of the various components, such as the active ingredient, may, for example, be contained in the medium at the exemplified concentrations at the start of culturing, or may be supplied to the medium after the start of culturing so as to reach the exemplified concentrations. Also, any of the various components, such as the active ingredient, may, for example, be contained in the medium at the exemplified concentrations at the start of culturing and also be further supplied to the medium after the start of culturing (e.g., after consumption of said component) so as to reach the exemplified concentrations.

The length of "a part of the culture period" is not particularly limited as long as the object of the present invention can be achieved (e.g., a growth-improving effect or a differentiation-promoting effect is obtained). The length of "a part of the culture period" can be appropriately set according to various conditions such as the type of animal cell and the length of the culture period. "A part of the period" may be, for example, a period of 10% or more, 20% or more, 30% or more, 40% or more, 50% or more, 60% or more, 70% or more, 80% or more, 90% or more, 95% or more, 97% or more, or 99% or more of the total culture period. "A part of the period" may also be, for example, a period of 0.5 days or more, 1 day or more, 2 days or more, 3 days or more, 4 days or more, 5 days or more, 6 days or more, 7 days or more, 8 days or more, 9 days or more, 10 days or more, 12 days or more, or 15 days or more.

The concentration of any of the various components, such as the active ingredient, in the medium may be set to the exemplified concentrations, for example, as an average value over a specific period during culturing. That is, "culturing is carried out in a medium containing a certain component at a certain concentration," "a certain component is contained in the medium during culturing at a certain concentration," or "the concentration of a certain component in the medium during culturing is a certain concentration" may mean that the average value of the concentration of said component in the medium over a specific period during culturing is within the range of said concentration. "The average value of the concentration of a certain component in the medium over a specific period during culturing" is not particularly limited as long as it can grasp the fluctuation of the concentration of said component during a specific period of culturing, but may mean, for example, the average value of the concentration of said component in the medium measured every 60 minutes, 30 minutes, 20 minutes, or 10 minutes over a specific period during culturing. Examples of "a specific period during culturing" include the entire culture period and a part of the culture period. "A part of the culture period" is as described above.

Any of the various components, such as the active ingredient, may be supplied to the medium throughout the entire culture period, or may be supplied to the medium only during a part of the culture period. "A part of the culture period" is as described above. Any of the various components, such as the active ingredient, may be supplied to the medium continuously, for example, or may be supplied to the medium intermittently. Any of the various components, such as the active ingredient, may be supplied to the medium daily, for example, or may be supplied to the medium every few days.

The concentration of any of the various components, such as the active ingredient, in the fed-batch or perfusion medium may be, for example, within the range of the concentration of said component in the medium exemplified above. The concentration of any of the various components, such as the active ingredient, in the fed-batch or perfusion medium may be, for example, 1-fold or more, 1.1-fold or more, 1.3-fold or more, 1.5-fold or more, 2-fold or more, 3-fold or more, 5-fold or more, 7-fold or more, 10-fold or more, 15-fold or more, or 20-fold or more the concentration of said component in the medium exemplified above, and may be 100-fold or less, 70-fold or less, 50-fold or less, 30-fold or less, 20-fold or less, 15-fold or less, 10-fold or less, 7-fold or less, 5-fold or less, 3-fold or less, or 2-fold or less the concentration, or any non-contradictory combination thereof. Specifically, the concentration of any of the various components, such as the active ingredient, in the fed-batch or perfusion medium may be, for example, 1 to 2 times, 1.1 to 2-fold, 1.3 to 2-fold, 1.5 to 2-fold, 2 to 3-fold, 3 to 5-fold, 5 to 7-fold, 7 to 10-fold, 10 to 15-fold, 15 to 20-fold, 20 to 30-fold, 20 to 50-fold, 20 to 70-fold, or 20 to 100-fold the concentration of said component in the medium exemplified above. Specifically, the concentration of any of the various components, such as the active ingredient, in the fed-batch or perfusion medium may be, for example, 1 to 100-fold, 2 to 50-fold, or 5 to 20-fold the concentration of said component in the medium exemplified above.

The concentration of any of the various components, such as the active ingredient, can be measured, for example, by a known method used for the detection or identification of compounds. Such methods include HPLC, UPLC, LC/MS, GC/MS, and NMR.

Animal cells can be cultured as described above. In one embodiment, a product (e.g., cultured meat or a target substance) may be obtained by culturing animal cells as described above. When a target substance is produced, the target substance may accumulate in the culture, for example, in the medium, on the cell surface, inside the cells, or a combination thereof. The target substance may be recovered from the culture as appropriate. That is, the method for producing the target substance may further include a step of recovering the target substance.

### <4> Use of the Active Ingredient

The present invention also discloses the use of the active ingredient for the applications exemplified above. That is, the present invention discloses, for example, the use of the active ingredient for culturing animal cells, the use of the active ingredient for improving the growth of animal cells, the use of the active ingredient for promoting the differentiation of animal cells, and the use of the active ingredient in the production of the composition of the present invention, such as a composition for animal cell culture.

The present invention also discloses an active ingredient for use in the applications exemplified above. That is, the present invention discloses, for example, an active ingredient for use in culturing animal cells, an active ingredient for use in improving the growth of animal cells, an active ingredient for use in promoting the differentiation of animal cells, and an active ingredient for use in the production of a composition for animal cell culture.

### EXAMPLES

Hereinafter, the present invention will be described more specifically with reference to nonlimiting examples.

### Example 1: Evaluation of the activity of hinokitiol using bovine muscle stem cells in a serum-free medium

### (1-1) Preparation of bovine muscle stem cells

Muscle tissue was collected from edible bovine shank meat, and CD56-positive cells were isolated using a Magnetic-Activated Cell Sorting (MACS) system to obtain bovine muscle stem cells.

### (1-2) Evaluation of the activity of hinokitiol using bovine muscle stem cells in a serum-free medium

The bovine muscle stem cells obtained in (1-1) were suspended in a serum medium. As the serum medium, a medium was used in which Advanced DMEM (Thermo Fisher Scientific, Cat No. 12491015) was supplemented with 20% FBS (Thermo Fisher Scientific, Cat No. 10270106), 10% horse serum (Thermo Fisher Scientific, Cat No. 16050-130), and 1% GlutaMAX^{™} Supplement (Thermo Fisher Scientific, Cat No. 35050061). The cell suspension was seeded into a 12-well plate (BD FALCON, Cat# 353046) coated with 1 µg/cm² fibronectin (Corning, Cat# F2006) at a cell density of 25,000 cells/0.2 mL, and the medium was changed every other day. On the fourth day of culture, the cells were subcultured into a hinokitiol-containing serum-free medium, a hinokitiol- and iron-containing serum-free medium, an iron-containing serum-free medium, a transferrin- and iron-containing serum-free medium, a transferrin-containing serum-free medium, or a serum-free medium not containing hinokitiol and transferrin, and the medium was changed every other day. As the base for all media, E8 medium (DMEM/F12, 64 mg/L I-ascorbic acid 2-phosphate magnesium, 14 µg/L sodium selenium, 19.4 mg/L insulin, 543 mg/L NaHCO₃, 2 µg/L TGFβ1, adjusted to pH 7.4 and 340 mOsm. Chen, G. et al. Nature Methods 8, 424-429 (2011)) supplemented with 5 ng/mL HGF (Peprotech, Cat# 294HGN005), 20 ng/mL EGF (Sigma, Cat# E9644), and 10 ng/mL bFGF (Peprotech, Cat#AF-450-62) was used. The E8 medium contains approximately 1.63 µM of iron. Table 1 summarizes the group numbers and media. Cell counting and subculturing were performed every 4 days, and the growth rate for two consecutive passages from day 4 to day 12 was calculated.

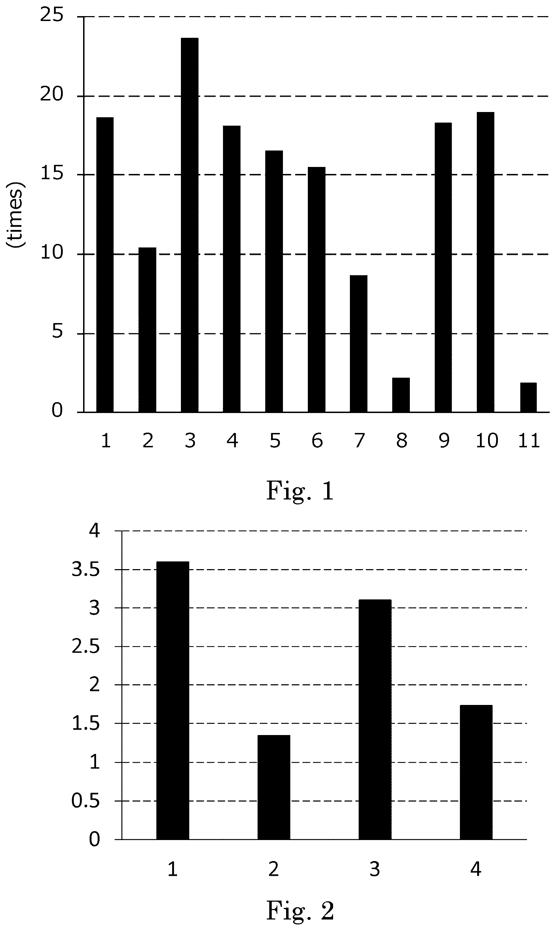

The results are shown in Fig. 1. A cell proliferation-promoting effect of hinokitiol on bovine muscle stem cells was observed. That is, it was revealed that an iron ionophore such as hinokitiol is effective for culturing animal cells (particularly for promoting proliferation). It was also revealed that transferrin can be replaced by an iron ionophore such as hinokitiol. Furthermore, in some cases, the cell proliferation-promoting effect of hinokitiol on bovine muscle stem cells was further improved by enhancing the iron concentration.

### [Table 1]

**Table 1**

| Group Number | Additives |
|---|---|
| 1 | hinokitiol 0.30 µM |
| 2 | hinokitiol 0.03 µM |
| 3 | hinokitiol 0.30 µM + FeSO₄ · 7H₂O 1.0 µM |
| 4 | hinokitiol 0.03 µM + FeSO₄ · 7H₂O 1.0 µM |
| 5 | hinokitiol 0.30 µM + FeSO₄ · 7H₂O 10.1 µM |
| 6 | hinokitiol 0.03 µM + FeSO₄ · 7H₂O 10.1 µM |
| 7 | FeSO₄ · 7H₂O 1.0 µM |
| 8 | FeSO₄ · 7H₂O 10.1 µM |
| 9 | transferrin 0.14 µM + FeSO₄ + 7H₂O 10.1 µM |
| 10 | transferrin 0.14 µM |
| 11 | distilled water |

### Example 2: Evaluation of the activity of hinokitiol using porcine subcutaneous preadipocytes (PSPA) in a serum-free medium

### (2-1) Acquisition of PSPA

As the porcine subcutaneous preadipocytes (PSPA), an adipose precursor cell line derived from porcine fetal subcutaneous tissue, described in Nakajima, et al. Biochem Biophys Res Commun. 2003 Sep 26;309(3):702-8, was obtained from RIKEN RCB and used.

### (2-2) Evaluation of the activity of hinokitiol using porcine subcutaneous preadipocytes (PSPA) in a serum-free medium

The porcine subcutaneous preadipocytes (PSPA) from (2-1) were cultured using a serum medium. As the serum medium, a medium was used in which DMEM (Gibco, Cat No. 10313-021) was supplemented with 10% FBS (Thermo Fisher Scientific, Cat No. 10270106), 1% GlutaMAX^{™} Supplement (Thermo Fisher Scientific, Cat No. 35050061), and 1% Penicillin-Streptomycin (Nacalai Tesque, Cat No. 09367-34). As the serum-free medium, a medium based on the aforementioned E8 medium was used. PSPA were seeded into a 12-well plate at a density of 10,000 cells/cm², and the cells were subcultured into a serum medium, a transferrin-containing serum-free medium, or a hinokitiol- and iron-containing serum-free medium (transferrin-free), with medium exchange performed every other day. Table 2 summarizes the group numbers, media, and additives. Cell counting was performed 4 days after the start of culturing, and the fold increase in cell number was calculated.

The results are shown in Fig. 2. A cell proliferation-promoting effect of hinokitiol on porcine subcutaneous preadipocytes was observed.

### [Table 2]

**Table 2**

| Group Number | Medium | Additives |
|---|---|---|
| 1 | Serum medium | - |
| 2 | Serum-free medium | transferrin 0.01 µM |
| 3 | Serum-free medium | hinokitiol 0.25 µM + FeSO₄ + 7H₂O 1 µM |
| 4 | Serum-free medium | hinokitiol 0.5 µM + FeSO₄ + 7H₂O 1 µM |

### Example 3: Confirmation of the proliferation of HEK293 cells by addition of hinokitiol using a suspension culture system

### (3-1) Acquisition of HEK293 cells

As HEK293, Viral Production Cells 2.0 (Thermo Fisher Scientific: A49784), which is a clonal cell line derived from the HEK293 cell line, was used. The HEK293 cell line is widely known as a cell line derived from human embryonic kidney.

### (3-2) Evaluation of the activity of hinokitiol using HEK293 cells in a suspension culture system

Using a 125 mL Erlenmeyer flask (VIOLAMO, SEF 125V), Viral Production Cells 2.0, a clonal cell line derived from the HEK293 cell line, were seeded into 30 mL of Viral Production Medium (Thermo Fisher Scientific: A4817901) supplemented with GlutaMAX^{™} Supplement (Thermo Fisher Scientific, Cat No. 35050061) to a final concentration of 4 mM. The cells were cultured with agitation in an incubator set at 37°C and 8% CO₂, and were subcultured twice. Thereafter, the cells were subcultured and cultured in a medium based on 30 mL of Basal 10 (Ajinomoto Healthy Supply, Co., Inc., CELLIST^{™} Basal media BASAL10) or an in-house medium supplemented with hinokitiol to a final concentration of 500 nM. The process of measuring the viable cell count every 3 or 4 days using a Vi-CELL BLU Cell Viability Analyzer (Beckman Coulter) before subculturing was repeated, and the cells were cultured for a total of 20 days.

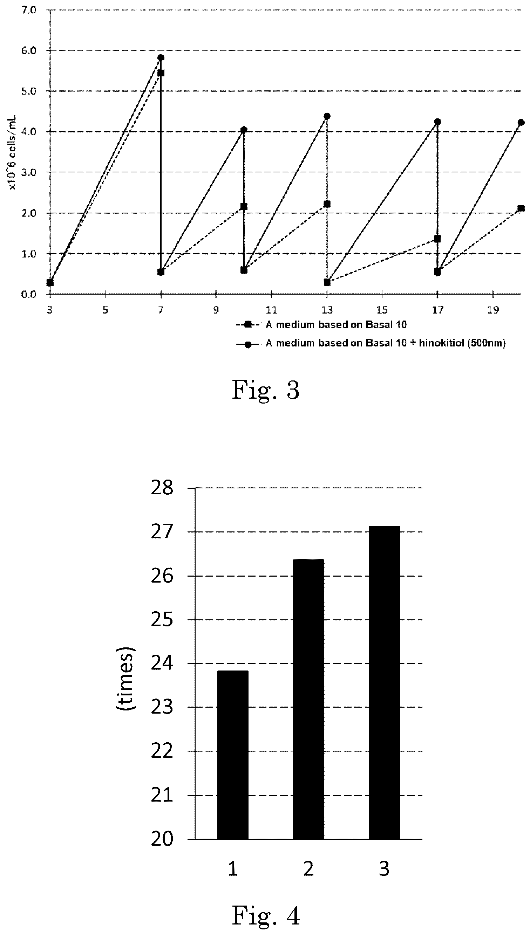

The results are shown in Fig. 3. A cell proliferation-promoting effect was observed in HEK293 cells due to the addition of hinokitiol.

### Example 4: Evaluation of the activity of hinokitiol using CD8-positive human T cells in a serum-free medium

### (4-1) Evaluation of the activity of hinokitiol using CD8-positive human T cells in a serum-free medium

CD3 Monoclonal Antibody (Thermo Fisher Scientific, Cat No. 16-0037-85) was mixed with PBS and added to a 24-well plate. After incubating at 37°C for 1 hour, the plate was washed 3 times with PBS. As a base medium, CTS^{™} OpTmizer^{™} T Cell Expansion (Thermo Fisher Scientific, Cat No. A104851) supplemented with CTS^{™} Immune Cell SR (Thermo Fisher Scientific, Cat No. A2596101), 200 mM L-glutamine (Thermo Fisher Scientific, Cat No. 25030081), P/S/Amphotericin B (Fujifilm-Wako, Cat No. 161-23181), and IL2 (PeproTech, Cat No. 200-02) was used. In the coated 24-well plates, medium groups with hinokitiol added as shown in Table 3 were prepared, and CD8+ T cells (LONZA, Cat No. 2W-300) were cultured in each respective group. A cell count was performed after 7 days, and the proliferation rate was calculated.

### [Table 3]

**Table 3**

| Group Number | Additives |
|---|---|
| 1 | distilled water |
| 2 | hinokitiol 0.49 µM |
| 3 | hinokitiol 0.0049 µM |

The results are shown in Fig. 4. A cell proliferation-promoting effect of hinokitiol on CD8-positive human T cells was observed.

### [INDUSTRIAL APPLICABILITY]

According to the present invention, animal cells can be cultured.

## Claims

1. A composition for animal cell culture, comprising an iron ionophore.

2. The composition according to claim 1, which is a composition for improving the growth and/or promoting the differentiation of animal cells.

3. The composition according to claim 1 or 2, which is a medium or a medium supplement.

4. The composition according to claim 3, wherein the medium is a basal medium, a fed-batch medium, or a perfusion medium.

5. The composition according to claim 1 or 2, wherein the iron ionophore is at least one component selected from the group consisting of hinokitiol, α-thujaplicin, and γ-thujaplicin.

6. The composition according to claim 1 or 2, wherein the iron ionophore is hinokitiol.

7. The composition according to claim 1 or 2, further comprising iron.

8. The composition according to claim 7, wherein the iron is an iron salt.

9. The composition according to claim 1 or 2, wherein the animal is a mammal, a bird, a fish, or a crustacean.

10. The composition according to claim 1 or 2, wherein the animal is an animal for meat production.

11. The composition according to claim 1 or 2, wherein the animal is a cow, a pig, or a chicken.

12. The composition according to claim 1 or 2, wherein the animal is a human.

13. The composition according to claim 1 or 2, wherein the cell is a muscle stem cell, an adipose precursor cell, a kidney-derived cell, or a T cell.

14. The composition according to claim 1 or 2, which is substantially free of serum.

15. The composition according to claim 1 or 2, which is substantially free of animal-derived albumin.

16. The composition according to claim 1 or 2, which is substantially free of transferrin.

17. The composition according to claim 1 or 2, which has a function of improving the growth of animal cells and/or a function of promoting the differentiation of animal cells.

18. A method for producing a product, comprising a step of culturing animal cells in the presence of an iron ionophore.

19. The method according to claim 17, wherein the product is cultured meat.

20. A method for culturing animal cells, comprising a step of culturing animal cells in the presence of an iron ionophore.

21. A method for improving the growth of animal cells, comprising a step of culturing animal cells in the presence of an iron ionophore.

22. A method for promoting the differentiation of animal cells, comprising a step of culturing animal cells in the presence of an iron ionophore.

23. The method according to any one of claims 18 to 22, wherein the iron ionophore is at least one component selected from the group consisting of hinokitiol, α-thujaplicin, and γ-thujaplicin.

24. The method according to any one of claims 18 to 22, wherein the iron ionophore is hinokitiol.

25. The method according to any one of claims 18 to 22, wherein the culturing is performed in the presence of iron.

26. The method according to claim 25, wherein the iron is an iron salt.

27. The method according to any one of claims 18 to 22, wherein the concentration of the iron ionophore in the medium during culturing is from 1 nM to 100 µM.

28. The method according to any one of claims 18 to 22, wherein the concentration of the iron ionophore in the medium during culturing is from 10 nM to 10 µM.

29. The method according to claim 24, wherein the concentration of hinokitiol in the medium during culturing is from 1 nM to 2.5 µM.

30. The method according to claim 24, wherein the concentration of hinokitiol in the medium during culturing is from 10 nM to 1 µM.

31. The method according to claim 25, wherein the concentration of iron in the medium during culturing is from 200 nM to 500 µM.

32. The method according to any one of claims 18 to 22, wherein the animal is a mammal, a bird, a fish, or a crustacean.

33. The method according to any one of claims 18 to 22, wherein the animal is an animal for meat production.

34. The method according to any one of claims 18 to 22, wherein the animal is a cow, a pig, or a chicken.

35. The method according to any one of claims 18, 20, 21, and 22, wherein the animal is a human.

36. The method according to any one of claims 18 to 22, wherein the cell is a muscle stem cell or an adipose precursor cell.

37. The method according to any one of claims 18, 20, 21, and 22, wherein the cell is a kidney-derived cell or a T cell.

38. The method according to any one of claims 18 to 22, wherein the culturing is performed in a medium substantially free of serum.

39. The method according to any one of claims 18 to 22, wherein the culturing is performed in a medium substantially free of animal-derived albumin.

40. The method according to any one of claims 18 to 22, wherein the culturing is performed in a medium substantially free of transferrin.

41. The method according to any one of claims 18 to 22, wherein by culturing the animal cells in the presence of the iron ionophore, the growth of the animal cells is improved and/or the differentiation of the animal cells is promoted.
